# EUROPEAN PATENT APPLICATION

(11) **EP 4 454 580 A1**
(43) Date of publication of application: **30.10.2024**
(21) Application number: 22910697.6
(22) Date of filing: 18.11.2022
(51) Int. Cl.: A61B 17/72, A61B 17/76

(54) **OSTEOSYNTHESIS IMPLEMENT, OSTEOSYNTHESIS SET, AND OSTEOSYNTHESIS MEMBER SET**

(30) Priority: 22.12.2021 JP 2021208193
(71) Applicant: Hoya Technosurgical Corporation, Tokyo 160-0004 (JP)
(72) Inventor: YANAGIDA Shun, Tokyo 160-0004 (JP); ONOSE Takeshi, Tokyo 160-0004 (JP)
(74) Representative: TBK
(86) International application number: PCT/JP2022/042833
(87) International publication number: WO 2023/119979

(57) **Abstract**

To provide an osteosynthesis implement (100), an osteosynthesis implement (100) set, and an osteosynthesis member set, capable of reliably restricting the rotation of an osteosynthesis member (201) after attachment to a fracture place of a patient.

An osteosynthesis implement (100) of the present invention is an osteosynthesis implement (100) to be inserted into a intramedullary nail (101), the osteosynthesis implement (100) including a shaft portion (211), and a screw engagement portion (221) formed so that a plurality of unit screw threads (222, 222A, 222C, 222D, 222E, 222F, 222G), provided around a circumferential surface of the shaft portion (211), are continuous in a spiral shape. The osteosynthesis implement (100) includes: a shaft hole (218) extending in an axial direction inside the shaft portion (211); a missing portion (223) formed by removing part of a specific unit screw thread (222A, 222C, 222D, 222F, 222G) so that a pair of screw grooves (224, 224A, 224B, 224C, 224D), adjacent across the specific unit screw thread (222A, 222C, 222D, 222F, 222G), face each other; and a hole portion (215) that is opened so that each screw groove of the pair of screw grooves (224, 224A, 224B, 224C, 224D) in a vicinity of the missing portion (223) communicates with the shaft hole (218) .

## Description

### Technical Field

The present invention relates to an osteosynthesis implement used for joining bones, an osteosynthesis set including the osteosynthesis implement, and an osteosynthesis member set.

### Background Art

Conventionally, as a method for treating fractures in the vicinity of the bone head of a bone such as the femur and the humerus, which have the bone heads, use of a surgical instrument (a bone fixing device) has been proposed. In such a method, a surgical instrument that includes an intramedullary nail (a nail) is inserted into the bone in an axial direction of the bone, and a bone fixing element (a lag screw) is inserted into the intramedullary nail. The bone fixing element has a distal portion that includes a bone engagement structure, such as a screw thread. The distal portion also has an opening portion that is opened to an internally extending channel and used to allow injection of a material (for example, a bone-reinforcing material) into the bone after implantation (see, for example, PTL1). The opening portion is provided in a screw groove between adjacent screw threads. The bone-reinforcing material extruded through the opening portion flows along the thread groove.

### Citation List

### Patent Literature

PTL 1: Japanese Patent No. 5931924

### Summary of Invention

### Technical Problem

In the bone fixing device in PTL 1, a material such as a bone-reinforcing material is hardened while filling the thread groove and spreading around the distal portion. At this time, the bone fixing element is constrained and fixed by the hardened material.

However, when part of the hardened material is damaged because of some reason and a gap is generated between the distal portion of the bone fixing element and the hardened material, the bone fixing element is less constrained, and thus the bone fixing element is easily rotated by vibrations caused by the motions of a patient. As a result, the bone fixing element cannot adequately immobilize the fractured portion of the patient.

At the time of removing the bone fixing element, it is necessary to apply a large rotational force to the bone fixing element when the bone fixing element is tightly constrained by the hardened material. However, application of an excessively large rotational force may destroy the bone, and thus removal of the bone fixing material is not easy.

In view of such circumstances, the present invention is intended to provide an osteosynthesis implement capable of reliably fixing a fracture portion of a patient, an osteosynthesis set including the osteosynthesis implement, and an osteosynthesis member set.

### Solution to Problem

An osteosynthesis implement of the present invention is an osteosynthesis implement to be inserted into a intramedullary nail, the osteosynthesis implement including a shaft portion, and a screw engagement portion formed so that a plurality of unit screw threads, provided around a circumferential surface of the shaft portion, are continuous in a spiral shape, the osteosynthesis implement including: a shaft hole extending in an axial direction inside the shaft portion; a missing portion formed by removing part of a specific unit screw thread so that internal spaces of a pair of screw grooves, adjacent across the specific unit screw thread, face each other; and a hole portion that is opened so that each screw groove of the pair of screw grooves in a vicinity of the missing portion communicates with the shaft hole.

In relation to the osteosynthesis implement of the present invention, the hole portion includes a long hole region formed so as to extend across both screw grooves in the pair thereof.

In relation to the osteosynthesis implement of the present invention, the long hole region extends in a direction that is approximately parallel to a length direction of the shaft portion.

In relation to the osteosynthesis implement of the present invention, the unit screw threads adjacent to the specific unit screw thread do not have a missing region facing the missing portion of the specific unit screw thread.

In relation to the osteosynthesis implement of the present invention, the missing portion and the hole portion are provided in a plurality of places in a circumference direction of the shaft portion.

In relation to the osteosynthesis implement of the present invention, the missing portion and the hole portion are provided in four places at approximately equal intervals around an axis of the shaft portion.

In relation to the osteosynthesis implement of the present invention, the missing portion and the hole portion are not provided in unit screw threads other than the specific unit screw thread.

In relation to the osteosynthesis implement of the present invention, the missing portion does not extend across two or more of the unit screw threads.

An osteosynthesis implement of the present invention is an osteosynthesis implement to be inserted into an intramedullary nail, the osteosynthesis implement including a shaft portion having a shaft hole, and a screw thread provided at a tip of the shaft portion, in which the shaft portion includes a hole portion of an elongated shape extending in a direction approximately parallel to a length direction of the shaft portion, and the hole portion penetrates from a surface of the shaft portion to the shaft hole.

An osteosynthesis implement set of the present invention is an osteosynthesis implement set, including: the osteosynthesis implement according to any of those described above; and an injector configured to allow injection of an intraosseous injection agent into a bone, in which the injector includes an injection nozzle configured to be insertable into the shaft hole, and a single injection port provided so as to correspond to the hole portion at a tip of the injection nozzle.

An osteosynthesis member set of the present invention is an osteosynthesis member set, including: an osteosynthesis member to be inserted into an intramedullary nail, the osteosynthesis member including a shaft portion, a screw thread provided spirally around a circumferential surface of the shaft portion, and a shaft hole extending in an axial direction inside the shaft portion; and an injector configured to allow injection of an intraosseous injection agent into a bone, in which the osteosynthesis member includes: a missing portion provided in a unit screw thread so that internal spaces of a pair of screw grooves, adjacent across the unit screw thread, face each other, the unit screw thread corresponding to one round of 360° of the screw thread; a hole portion provided in the shaft portion so as to communicate with the missing portion; and an osteosynthesis member-side positioning portion, the injector includes: an injection nozzle configured to be insertable into the shaft hole of the osteosynthesis member; a single injection port provided at a tip portion of the injection nozzle; and an injector-side positioning portion, and the osteosynthesis member-side positioning portion and the injector-side positioning portion are used to position the injector with respect to the osteosynthesis member to allow the injection port to correspond to the hole portion.

### Advantageous Effects of Invention

The osteosynthesis implement, the osteosynthesis implement set, and the osteosynthesis member set of the present invention can demonstrate an excellent effect of reliably fixing the fracture place of a patient.

### Brief Description of Drawings

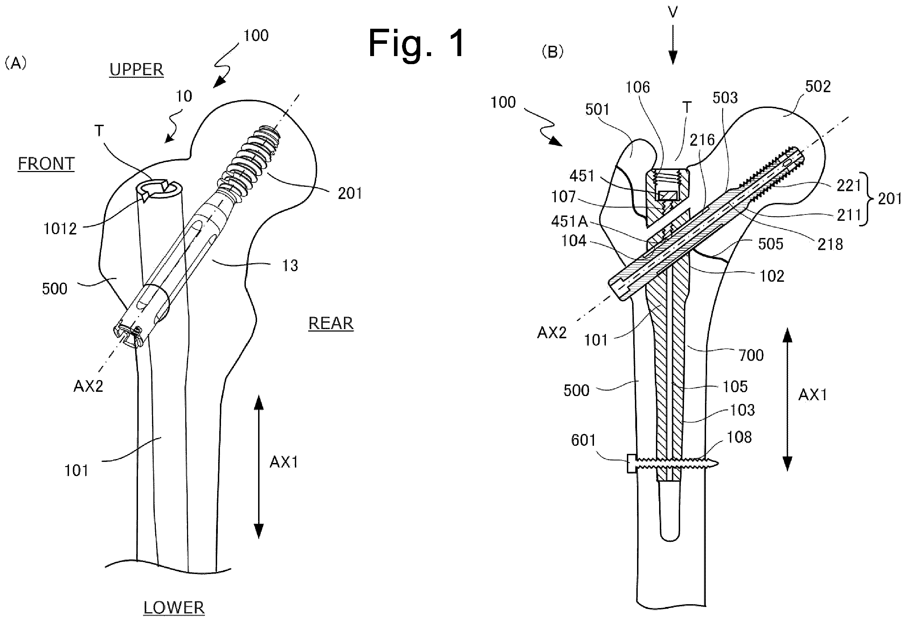

[Fig. 1] Fig. 1(A) is a perspective view showing a state in which an osteosynthesis implement according to a first embodiment of the present invention is attached to a fracture place of a patient mounted with, and Fig. 1(B) is a cross-sectional view of Fig. 1(A).

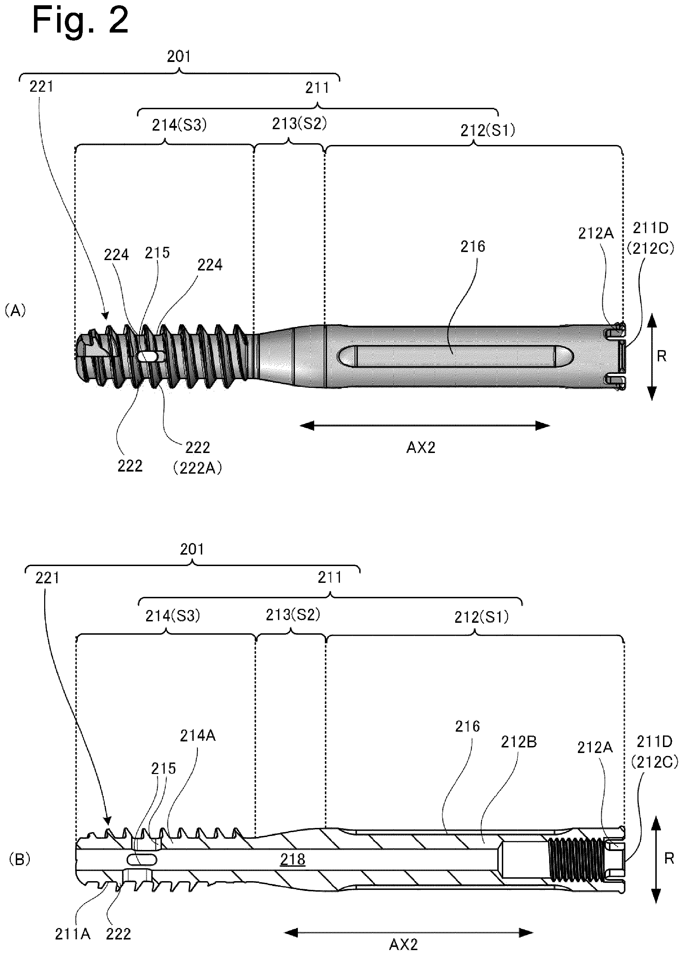

[Fig. 2] Fig. 2(A) is a view showing an osteosynthesis member in the first embodiment of the present invention, and Fig.2(B) is a cross-sectional view of the osteosynthesis member cut in an axial direction.

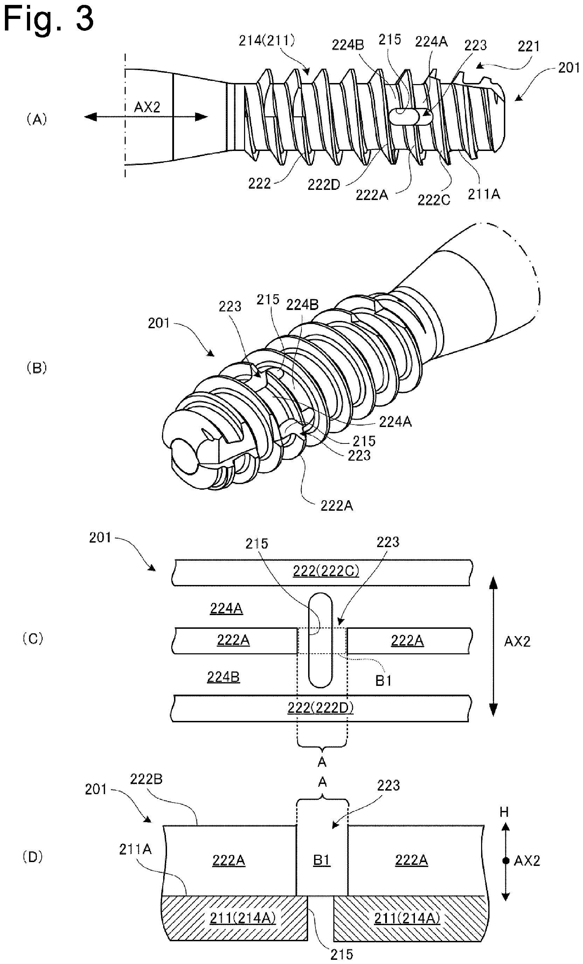

[Fig. 3] Fig. 3(A) is an enlarged plan view of a region including a screw engagement portion of the osteosynthesis member in the first embodiment of the present invention, Fig. 3(B) is a perspective view of the region including the screw engagement portion of the osteosynthesis member in the first embodiment of the present invention, Fig. 3(C) is an enlarged schematic plan view of a region including a missing portion of the osteosynthesis member in the first embodiment of the present invention, and Fig. 3(D) is an enlarged schematic cross-sectional view of the region including the missing portion of the osteosynthesis member in the first embodiment of the present invention.

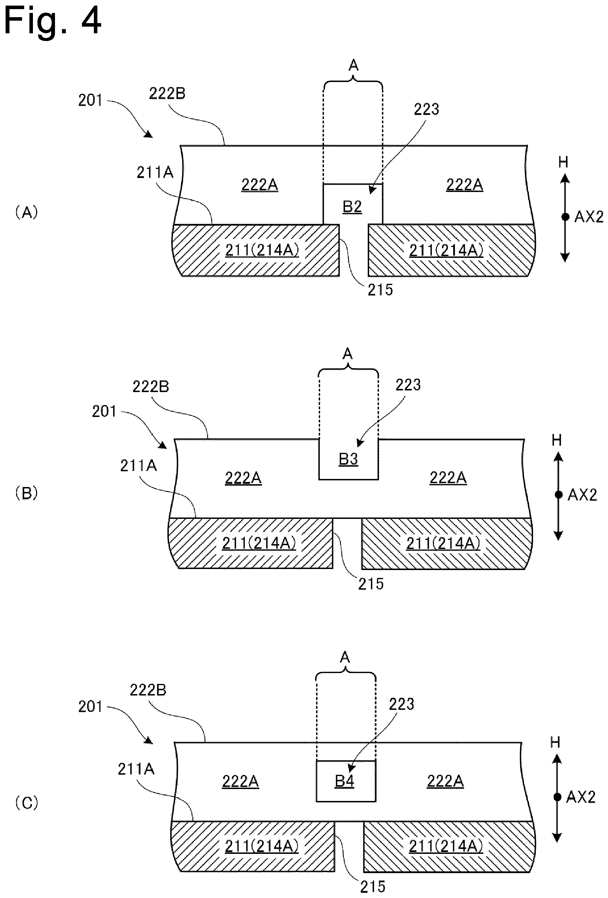

[Fig. 4] Figs. 4(A) to 4(C) are enlarged schematic cross-sectional views of a region including a missing portion in a modification of the missing portion of the osteosynthesis member in the first embodiment of the present invention.

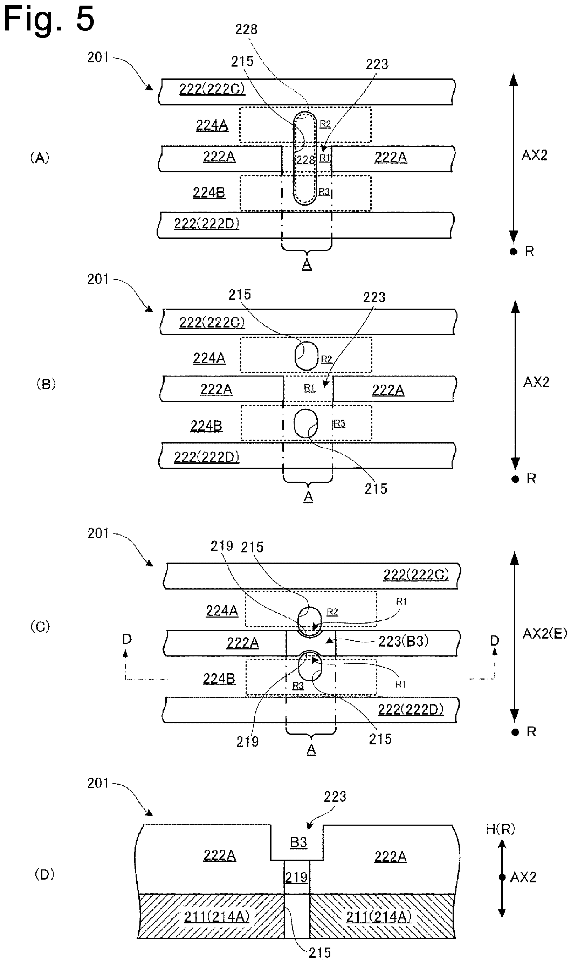

[Fig. 5] Figs. 5(A) to 5(C) are enlarged schematic plan views of a region including a modification of a hole portion of the osteosynthesis member in the first embodiment of the present invention, and Fig. 5(D) is a schematic cross-sectional view of Fig. 5(C) taken along a D-D arrow line.

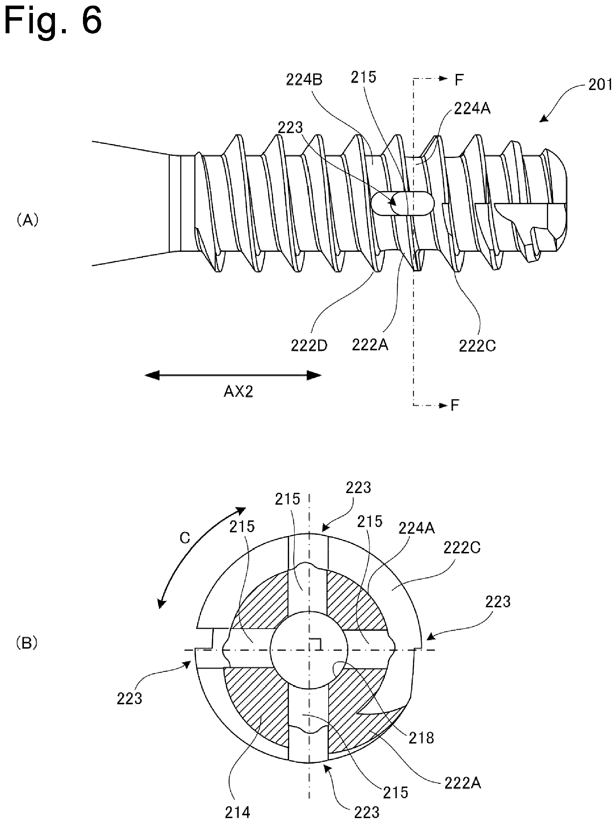

[Fig. 6] Fig. 6(A) is an enlarged plan view of the region including the screw engagement portion of the osteosynthesis member in the first embodiment of the present invention, and Fig. 6(B) is a cross-sectional view of Fig. 6(A) taken along an F-F arrow line.

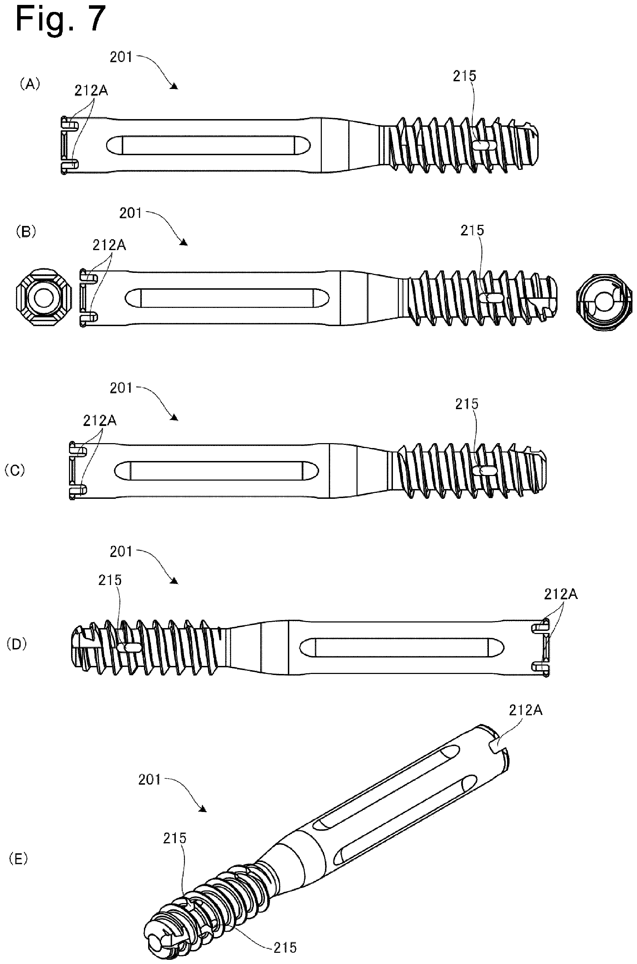

[Fig. 7] Fig. 7(A) is a plan view of the osteosynthesis member in the first embodiment of the present invention, Fig. 7(B) includes, in order from the left side, a left side view, a front view, and a right side view of the osteosynthesis member in the first embodiment of the present invention, Fig. 7(C) is a bottom view of the osteosynthesis member in the first embodiment of the present invention, Fig. 7(D) is a rear view of the osteosynthesis member in the first embodiment of the present invention, and Fig. 7(E) is a perspective view of the osteosynthesis member in the first embodiment of the present invention.

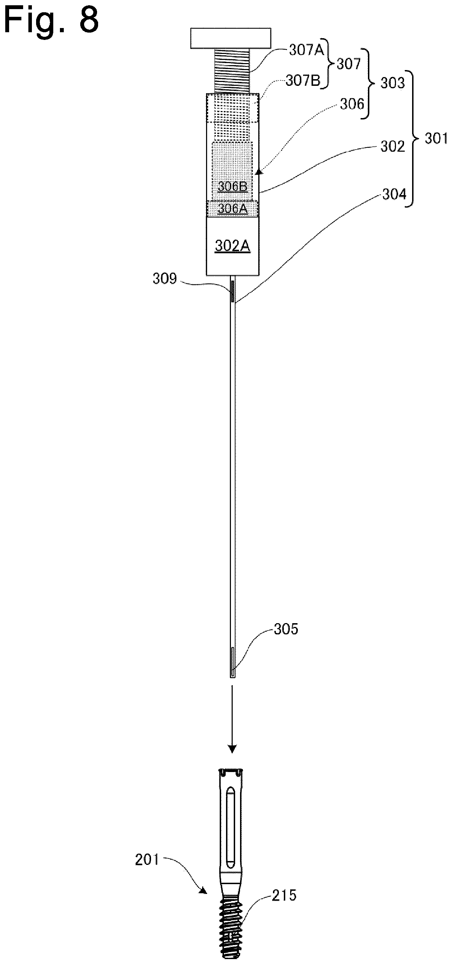

[Fig. 8] Fig. 8 is a schematic view showing an intraosseous injection agent insertion tool of an osteosynthesis implement set in the first embodiment of the present invention.

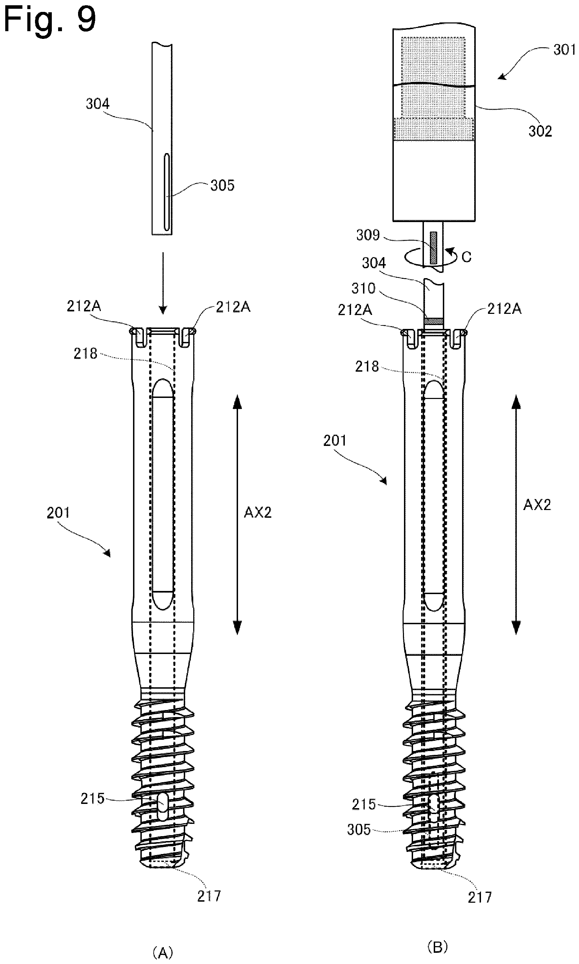

[Fig. 9] Fig. 9(A) is a schematic view showing the intraosseous injection agent insertion tool immediately before insertion into the osteosynthesis member in the first embodiment of the present invention, and Fig. 9(B) is a schematic view showing the intraosseous injection agent insertion tool inserted into the osteosynthesis member in the first embodiment of the present invention.

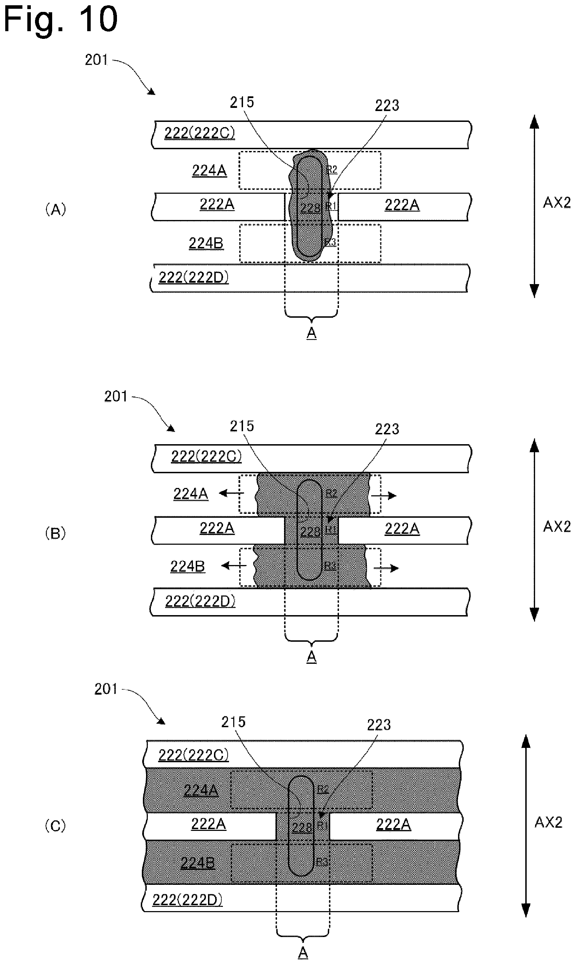

[Fig. 10] Figs 10(A) to 10(C) are enlarged schematic plan views showing, in chronological order, states in which the intraosseous injection agent extruded from the intraosseous injection agent insertion tool starts to spread through the hole portion to the surface of the shaft portion in the first embodiment of the present invention.

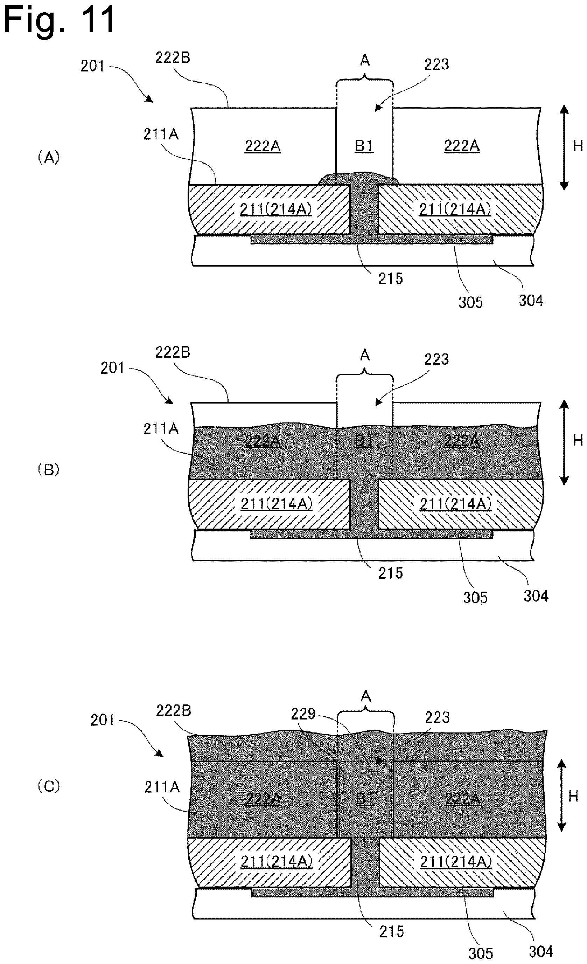

[Fig. 11] Figs 11(A) to (C) are enlarged schematic cross-sectional views showing, in chronological order, states in which the intraosseous injection agent extruded from the intraosseous injection agent insertion tool starts to spread through the hole portion to the surface of the shaft portion in the first embodiment of the present invention.

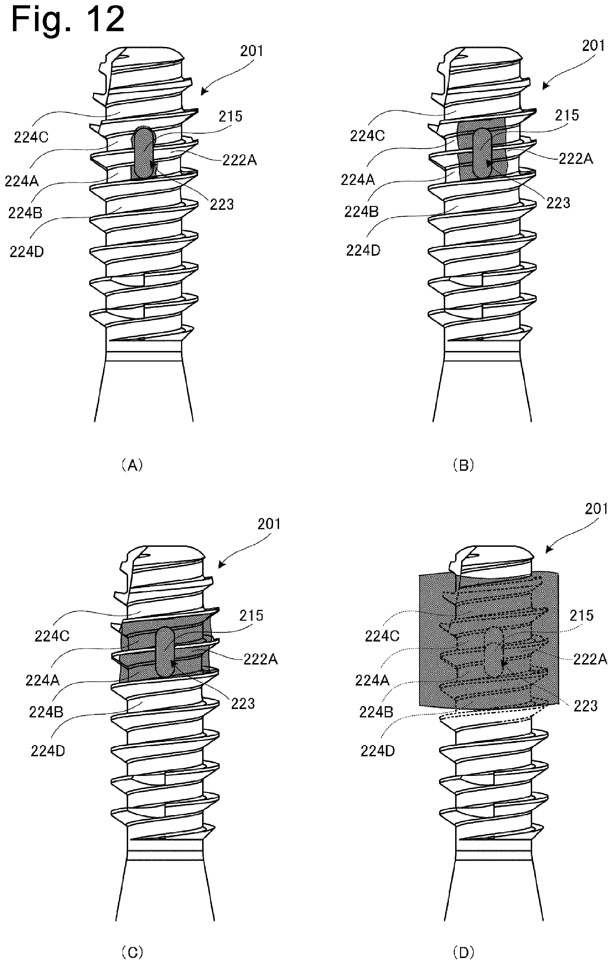

[Fig. 12] Figs 12(A) to 12(D) are enlarged front views showing, in chronological order, states in which the intraosseous injection agent extruded from the intraosseous injection agent insertion tool starts to spread through the hole portion to the surface of the shaft portion in the first embodiment of the present invention.

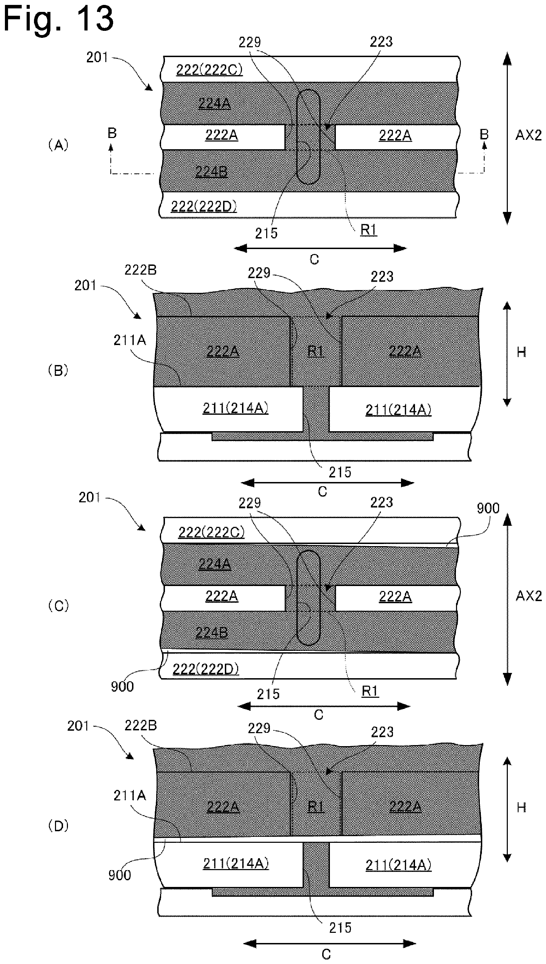

[Fig. 13] Fig. 13(A) is an enlarged schematic plan view showing the intraosseous injection agent filled in a region including the missing portion and the hole portion of the osteosynthesis member in the first embodiment of the present invention, Fig. 13(B) is a schematic cross-sectional view of Fig. 13(A) taken along a B-B arrow line, Fig. 13(C) is an enlarged schematic plan view showing a state in which a gap is generated between the intraosseous injection agent and the osteosynthesis member in a similar state as in Fig. 13(A), and Fig. 13(D) is an enlarged schematic cross-sectional view showing a state in which a gap is generated between the intraosseous injection agent and the osteosynthesis member in a similar state as in Fig. 13(B).

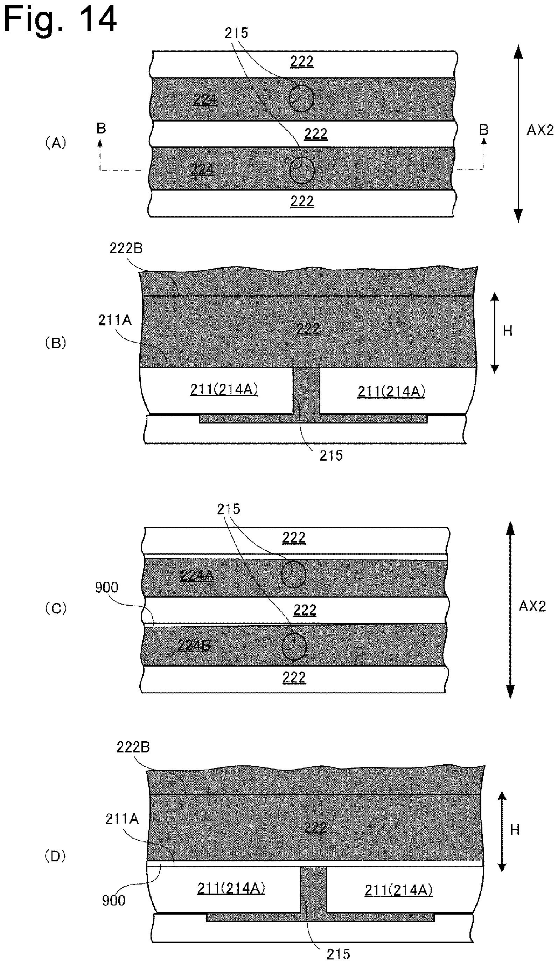

[Fig. 14] Fig. 14(A) is an enlarged schematic plan view showing a state in which the intraosseous injection agent is filled in a region including the hole portion of a conventional osteosynthesis member, Fig. 14(B) is a schematic cross-sectional view of 14(A) taken along a B-B arrow line, Fig. 14(C) is an enlarged plan view showing a state in which a gap is generated between the intraosseous injection agent and the osteosynthesis member in a similar state as in Fig. 14(A), and Fig. 14(D) is an enlarged schematic cross-sectional view showing a state in which a gap is generated between the intraosseous injection agent and the osteosynthesis member in a similar state as in Fig. 14(B).

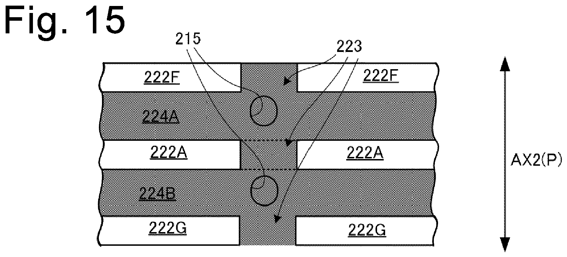

[Fig. 15] Fig. 15 is an enlarged plan view of the osteosynthesis member with three missing portions continuously provided in adjacent unit screw threads, as a modification of the osteosynthesis member in the first embodiment of the present invention.

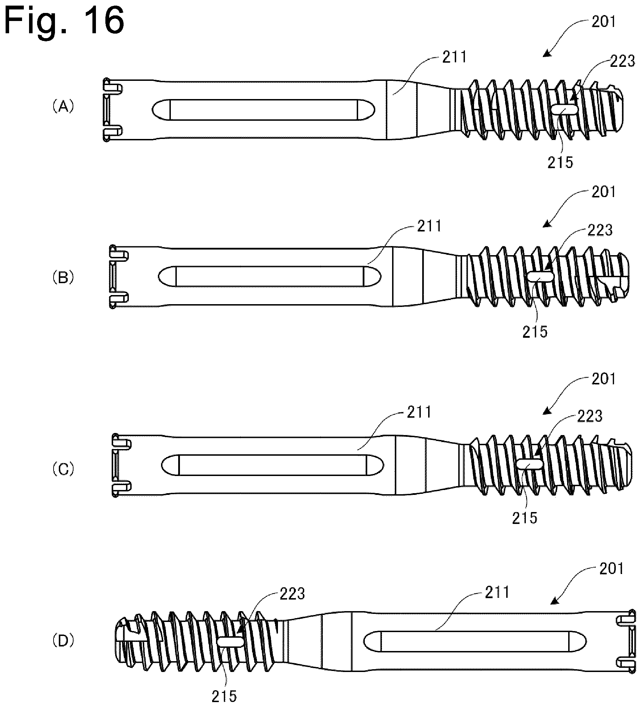

[Fig. 16] Fig. 16(A) is a plan view of an osteosynthesis member in a second embodiment of the present invention, Fig. 16(B) is a front view of the osteosynthesis member in the second embodiment of the present invention, Fig. 16(C) is a bottom view of the osteosynthesis member in the second embodiment of the present invention, and Fig. 16(D) is a rear view of the osteosynthesis member in the second embodiment of the present invention.

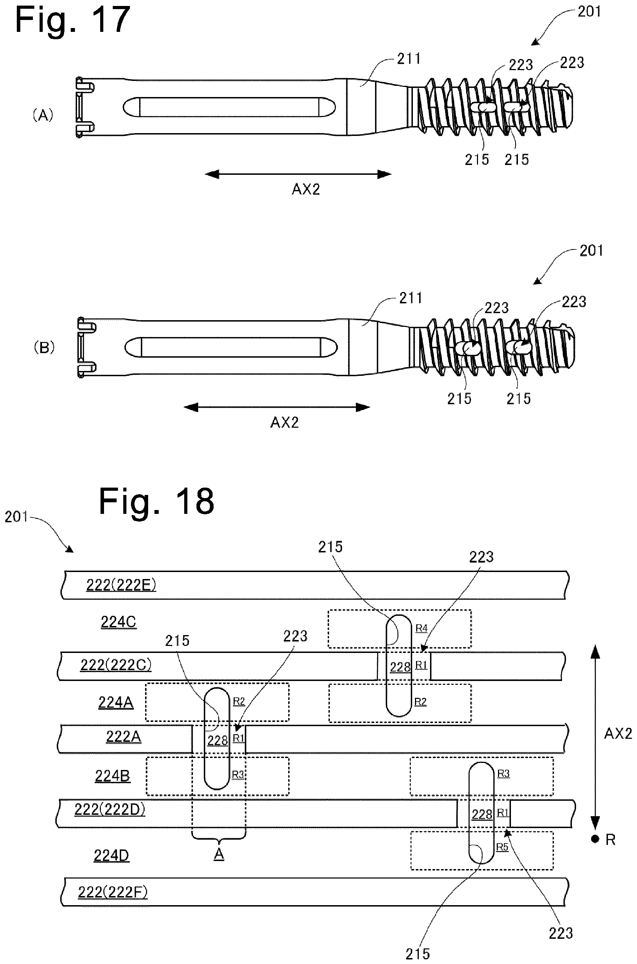

[Fig. 17] Fig. 17(A) is a plan view of an osteosynthesis member in a third embodiment of the present invention, and Fig. 17(B) is a front view of a modification of the osteosynthesis member in the third embodiment of the present invention.
[Fig. 18] Fig. 18 is a schematic view showing an osteosynthesis member in a fourth embodiment of the present invention.

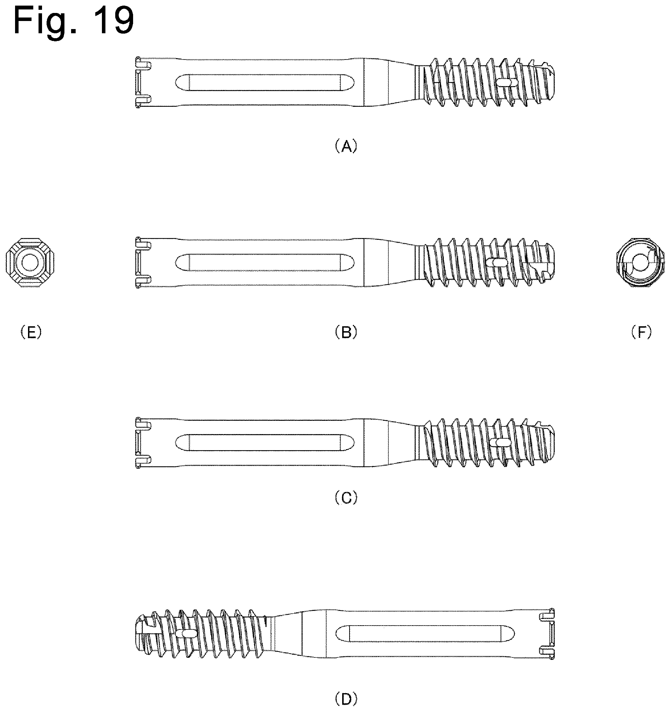

[Fig. 19] Fig. 19(A) is a right side view of the osteosynthesis member in the first embodiment of the present invention, Fig. 19(B) is a front view of the osteosynthesis member in the first embodiment of the present invention, Fig. 19(C) is a left side view of the osteosynthesis member in the first embodiment of the present invention, Fig. 19(D) is a rear view of the osteosynthesis member in the first embodiment of the present invention, Fig. 19(E) is a plan view of the osteosynthesis member in the first embodiment of the present invention, and Fig. 19(F) is a bottom view of the osteosynthesis member in the first embodiment of the present invention.

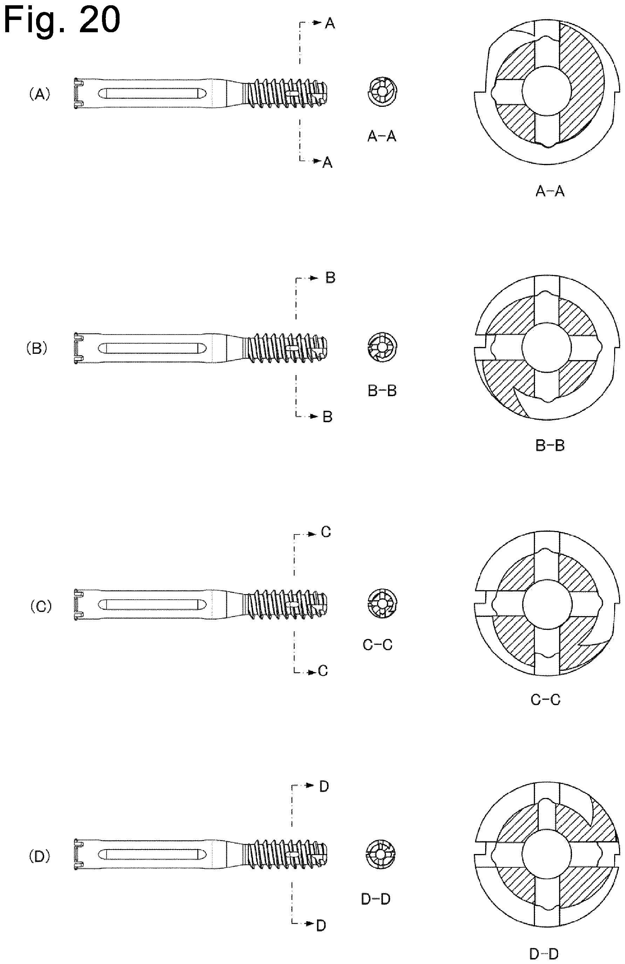

[Fig. 20] Fig. 20(A) includes a right side view of the osteosynthesis member in the first embodiment of the present invention provided on the left side, a cross-sectional view of the right side view taken along an A-A arrow line provided in the middle, and an enlarged cross-sectional view of the right side view taken along the A-A arrow line provided on the right side, Fig. 20(B) includes a front view of the osteosynthesis member in the first embodiment of the present invention provided on the left side, a cross-sectional view of the front view taken along a B-B arrow line provided in the middle, and an enlarged cross-sectional view of the front view taken along the B-B arrow line provided on the right side, Fig. 20(C) includes a left side view of the osteosynthesis member in the first embodiment of the present invention provided on the left side, a cross-sectional view of the left side view taken along a C-C arrow line provided in the middle, and an enlarged cross-sectional view of the left side view taken along the C-C arrow line provided on the right side, and Fig. 20(D) includes a rear view of the osteosynthesis member in the first embodiment of the present invention, a cross-sectional view of the rear view taken along a D-D arrow line provided in the middle, and an enlarged cross-sectional view of the rear view taken along the D-D arrow line provided on the right side.

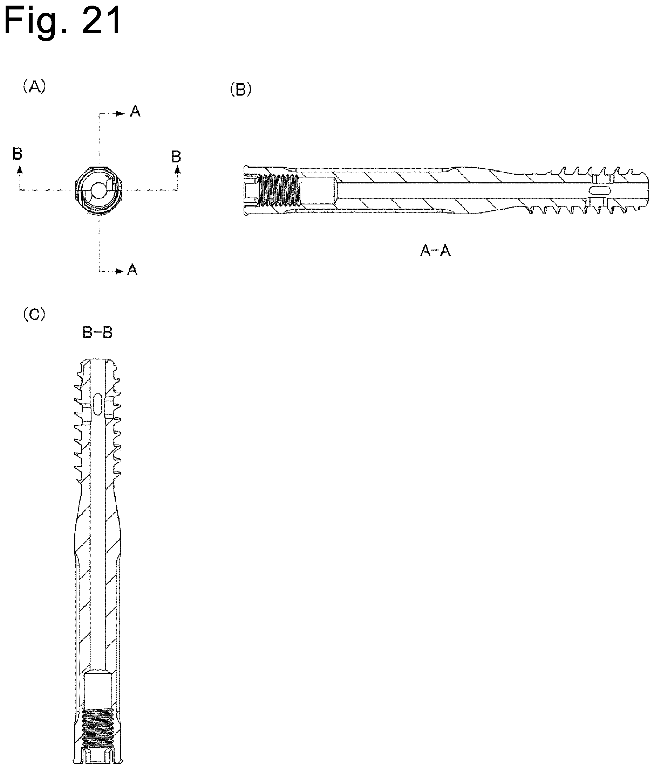

[Fig. 21] Fig. 21(A) is a bottom view of the osteosynthesis member in the first embodiment of the present invention, Fig. 21(B) is a cross-sectional view of Fig. 21(A) taken along an A-A arrow line, and Fig. 21(C) is a cross-sectional view of Fig. 21(A) taken along a B-B arrow line.

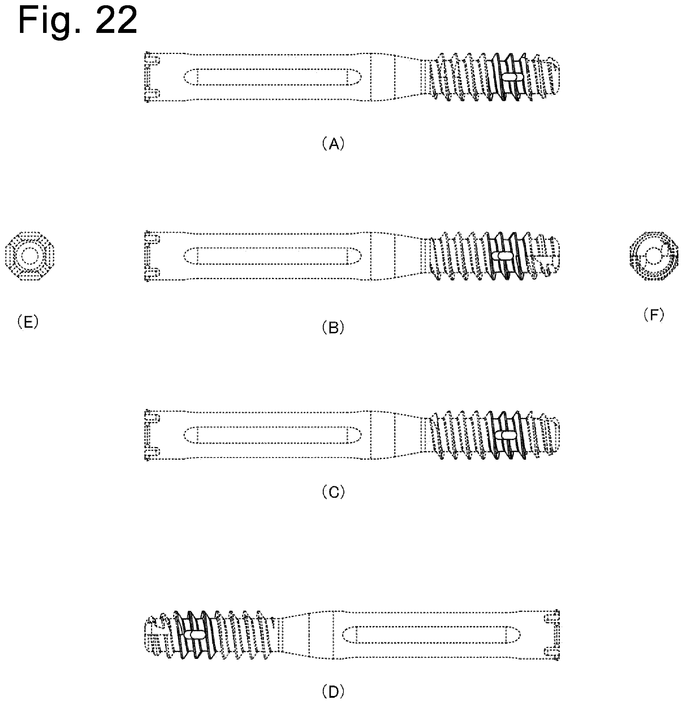

[Fig. 22] Fig. 22(A) is a right side view of the osteosynthesis member in the first embodiment of the present invention, Fig. 22(B) is a front view of the osteosynthesis member in the first embodiment of the present invention, Fig. 22(C) is a left side view of the osteosynthesis member in the first embodiment of the present invention, Fig. 22(D) is a rear view of the osteosynthesis member in the first embodiment of the present invention, Fig. 22(E) is a plan view of the osteosynthesis member in the first embodiment of the present invention, and Fig. 22(F) is a bottom view of the osteosynthesis member in the first embodiment of the present invention. Note that in the present invention, if there are portions to be registered separately as a partial design, they are expressed by solid lines, and other portions are expressed by broken lines. Dashed-dotted lines shown in the drawings express only the boundary between the portions to be registered as a partial design and the other portions.

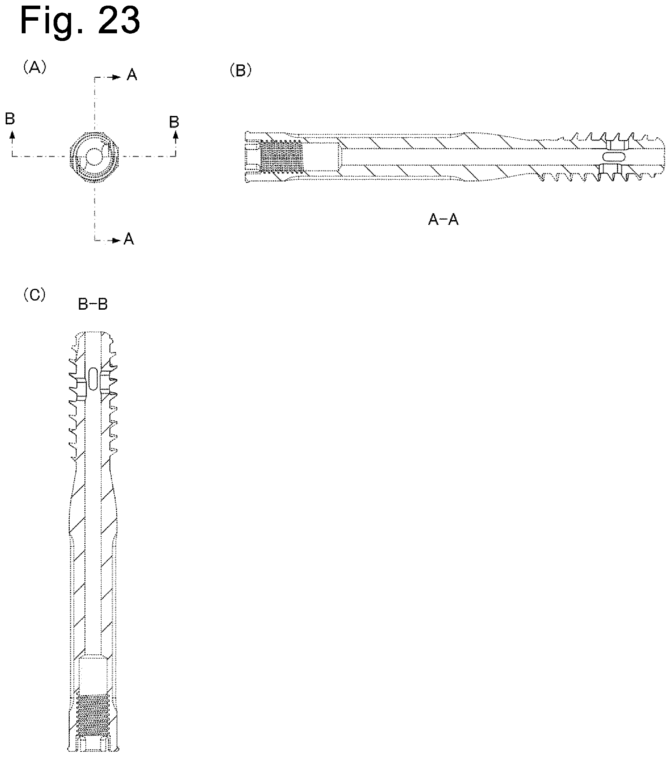

[Fig. 23] Fig. 23(A) is a bottom view of the osteosynthesis member in the first embodiment of the present invention, Fig. 23(B) is a cross-sectional view of Fig. 23(A) taken along an A-A arrow line, and Fig. 23(C) is a cross-sectional view of Fig. 23(A) taken along a B-B arrow line. Note that in the present invention, if there are portions to be registered separately as a partial design, they are expressed by solid lines, and other portions are expressed by broken lines.

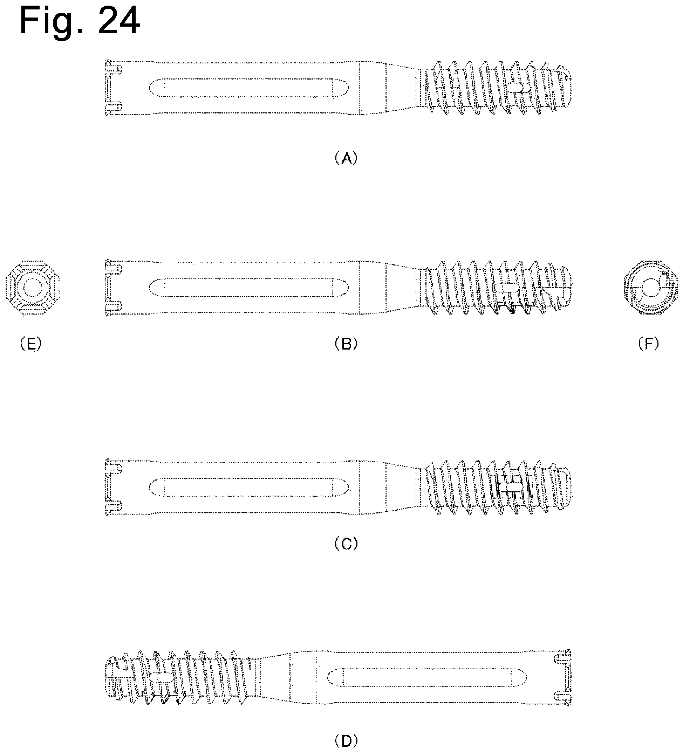

[Fig. 24] Fig. 24(A) is a right side view of the osteosynthesis member in the first embodiment of the present invention, Fig. 24(B) is a front view of the osteosynthesis member in the first embodiment of the present invention, Fig. 24(C) is a left side view of the osteosynthesis member in the first embodiment of the present invention, Fig. 24(D) is a rear view of the osteosynthesis member in the first embodiment of the present invention, Fig. 24(E) is a plan view of the osteosynthesis member in the first embodiment of the present invention, and Fig. 24(F) is a bottom view of the osteosynthesis member in the first embodiment of the present invention. Note that in the present invention, if there are portions to be registered separately as a partial design are expressed by solid lines, and other portions are expressed by broken lines. Dashed-dotted lines shown in the drawings express only the boundary between the portions to be registered as a partial design and the other portions.

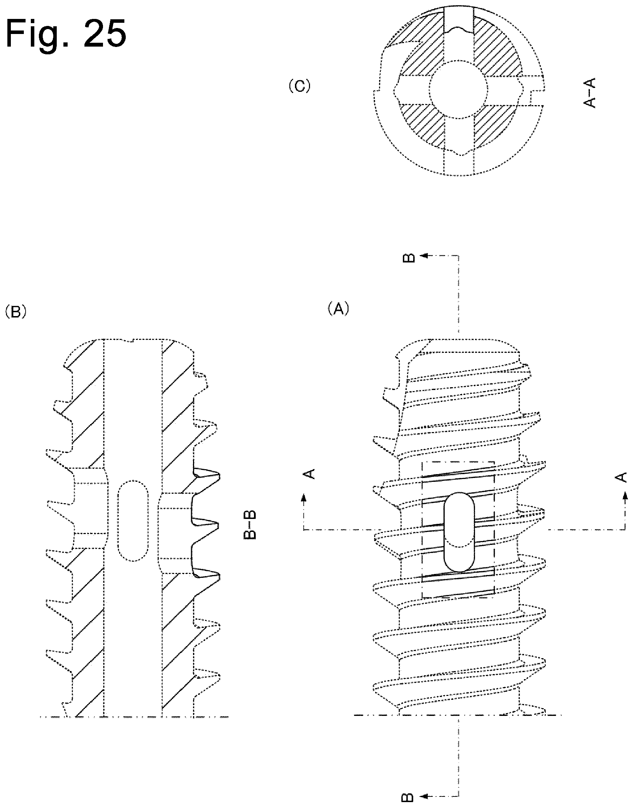

[Fig. 25] Fig. 25(A) is an enlarged view of a tip side of Fig. 25(C), Fig. 25(B) is a cross-sectional view of Fig. 25(A) taken along a B-B arrow line, and Fig. 25(C) is a cross-sectional view of Fig. 25(A) taken along an A-A arrow line. Description of Embodiments

Embodiments of the present invention will be described below with reference to the accompanying drawings. In each of the following drawings, portions with identical reference signs represent the identical component members. In each of the drawings, some component members are omitted as appropriate to simplify the drawings, and the size, shape, thickness, or the like of the members are exaggerated as appropriate.

### <First embodiment>

First, the entire configuration of an osteosynthesis implement 100 of a first embodiment of the present invention is described with reference to Fig. 1. Fig. 1 includes schematic views of a fracture place of a femur 500 of a patient fixed by the osteosynthesis implement 100, in which Fig. 1(A) is a perspective view showing an appearance partially in a perspective manner, and Fig.1(B) is a longitudinal cross-sectional view showing part of the osteosynthesis implement 100. Here, in the following description, unless otherwise stated, the direction is specified based on the state in which a fracture site of the femur 500 of the patient is fixed with the osteosynthesis implement 100. Specifically, the berry side of the patient is referred to as "front", the back side as "rear", the head side of the patient as "upper", and the leg side of the patient as "lower". Fig. 1 shows the femur 500 of a left leg as an example.

As shown in Fig. 1(A), the osteosynthesis implement 100 in the present embodiment includes, for example, an intramedullary nail (nail) 101, an osteosynthesis member (lag screw) 201, and an intramedullary nail aid 10. The intramedullary nail aid 10 in the present embodiment is selectively attachable to, for example, a conventionally known intramedullary nail 101. In other words, in the present embodiment, known intramedullary nails and osteosynthesis members can be adopted as the intramedullary nail 101 and the osteosynthesis member 201. The intramedullary nail aid 10 is attached to one end (an upper end T (base end) side) of the intramedullary nail 101 in an axis AX1 direction, the intramedullary nail 101 being a rod-shaped member formed into a shaft-like shape as a whole. In Fig. 1, the axis AX1 direction of the intramedullary nail 101 is an up-down direction in the drawing.

### <Intramedullary nail (nail)>

The intramedullary nail 101 will be described with reference to Fig. 1(B). Here, in Fig. 1(B), illustration of the intramedullary nail aid 10 is omitted.

The intramedullary nail 101 is inserted into the medullary cavity (internal cavity) of the femur 500 and used. The intramedullary nail 101 includes a proximal portion 102 that is on the base end (upper end T) side in the axis AX1 direction and a distal portion 103 located on the tip (lower end) side. The intramedullary nail 101 includes a shaft hole 105. The shaft hole 105 communicates with the proximal portion 102 and the distal portion 103, extends in the axis AX1 direction, and opens at both the ends in the axis AX1 direction. In the vicinity of the opening on the side of the proximal portion 102 (upper end T) of the shaft hole 105, a female screw 106 is formed on an inner wall (inner surface) of the shaft hole 105. Here, the shaft hole 105 may be provided only on the side of the proximal portion 102 of the intramedullary nail 101, and does not need to communicate with the distal portion 103. In other words, the distal portion 103 may be solid.

The intramedullary nail 101 includes a transverse hole 104 extending in a direction in which the transverse hole 104 intersects the shaft hole 105. The transverse hole 104 is a hole that allows insertion of the osteosynthesis member (lag screw) 201. The shaft hole 105 is open at both the upper and lower ends of the intramedullary nail 101, and is also open to the transverse hole 104 in the middle of the upper and lower ends. At this position, the shaft hole 105 and the transverse hole 104 communicate with each other. In this connected portion, and more specifically, in the shaft hole 105 above the transverse hole 104, a set screw 451 is inserted as a fixture of the osteosynthesis member 201.

In a cross-sectional view shown in Fig. 1(B), the transverse hole 104 diagonally penetrates the intramedullary nail 101 so as to have an axis AX2 that is inclined to the axis AX1 direction of the intramedullary nail 101. Hereafter, the axis AX2 direction of the transverse hole 104 is also called a transverse hole axial direction.

An inclination angle of the transverse hole 104 (its axis AX2) in Fig. 1(B) is appropriately set according to an application site (bone condition, fracture condition, etc.) of the osteosynthesis implement 100 so that when the intramedullary nail 101 is inserted into the medullary cavity of the femur 500, the osteosynthesis member 201 can fix the fracture site in the vicinity of a bone head 502 of the femur 500. As an example in the present embodiment, the inclination angle is set so as to approximately coincide with an angle formed between the axial direction (the up-down direction in the drawing) of the femur 500 and the direction of the bone head 502 protruding from the femur 500.

In this example, the intramedullary nail 101 also includes a fixture insertion hole 108 that penetrates the distal portion 103, with both the ends of the fixture insertion hole 108 being open to both the sides of the outer circumferential surface of the distal portion 103. Note that the fixture insertion hole 108 is formed so as to penetrate the distal portion 103 in a direction that is approximately orthogonal to the axis AX1 direction. The fixture insertion hole 108 allows insertion of a screw 601 or the like, and this insertion also fixes the intramedullary nail 101 and the femur 500 also in the distal portion 103.

### <Osteosynthesis member (Lag screw)>

Referring to Figs. 1 to 7, the osteosynthesis member (lag screw) 201 according to the present embodiment will be described in detail. As shown in Fig. 1(B), the osteosynthesis member (lag screw) 201, which is a rod-shaped member formed into a shaft-like shape as a whole, is inserted into the transverse hole 104 of the intramedullary nail 101. In other words, the axis AX2 direction of the osteosynthesis member 201 coincides with the axis AX2 direction (transverse hole axial direction) of the transverse hole 104.

As shown in Fig. 2(A), the osteosynthesis member 201 includes a shaft portion 211, and a screw engagement portion 221 provided in a prescribed section at the tip side of the shaft portion 211. As shown in Fig. 1(B), the screw engagement portion 221 is screwed into the bone head 502 along with the shaft portion 211 so as to fix the fracture site in the vicinity of the bone head 502. Here, the tip side of the shaft portion 211 refers to the side that is screwed into the bone head 502. The base end side of the shaft portion 211 refers to an end portion side opposite to the tip side of the shaft portion 211.

### <Shaft portion>

The shaft portion 211 will be described below with reference to Figs. 1 and 2. As shown in Fig. 1(B), the shaft portion 211 is a rod-like tubular body to be inserted into the transverse hole 104. As shown in Fig. 2(A), in the present embodiment, the shaft portion 211 is different in outer diameter (maximum outer diameter) or cross-sectional area for each section in the axis AX2 direction, and includes a large diameter portion 212 in a base end-side section S1 of the shaft portion 211, a tapered portion 213 in an intermediate section S2 of the shaft portion 211, and a small diameter portion 214 in a tip-side section S3 of the shaft portion 211. The large diameter portion 212, the tapered portion 213, and the small diameter portion 214 are connected in this order in the axis AX2 direction.

With the base end of the shaft portion 211 as a starting point, the large diameter portion 212, which is a portion larger in terms of the outer diameter (maximum outer diameter) or cross-sectional area than the small diameter portion 214, constitutes the base end-side section S1 of the shaft portion 211. Here, the cross-sectional area described above refers to the area of a cross section of the shaft portion 211 taken in a direction orthogonal to the axis AX2 direction. The tapered portion 213, which is configured to become gradually smaller in terms of the outer diameter from the base end side of the shaft portion 211 toward the tip side, constitutes the intermediate section S2 of the shaft portion 211 with a boundary with the large diameter portion 212 as a starting point. The small diameter portion 214 constitutes the tip-side section S3 of the shaft portion 211 with a boundary with the tapered portion 213 as a starting point. Incidentally, the tapered portion 213 is continuous to the large diameter portion 212 on the base end side, which is larger in outer diameter or cross-sectional area, and is also continuous to the small diameter portion 214 on the tip side, which is smaller in outer diameter or cross-sectional area.

As shown in Figs. 2(B) and 2(B), the large diameter portion 212 includes groove portions 216 that are recessed in a radial direction R of the shaft portion 211 with the outer circumferential surface as a starting point. The plurality of groove portions 216 are spaced apart in the circumferential direction around the axis of the shaft portion 211. In the present embodiment, four groove portions 216 are provided at equal intervals (every 90 degrees) in the circumferential direction around the axis of the shaft portion 211. As shown in Fig. 1(B), the groove portions 216 are brought into contact with, fitted to and/or pressed by a tip 451A of the set screw 451, so that the osteosynthesis member 201 in the state of being inserted into the transverse hole 104 is prevented from rotating, and is also fixed to the intramedullary nail 101.

Moreover, as shown in Figs. 2(A) and 2(B), at the base end of the large diameter portion 212, engagement recess portions 212A are provided for fitting a tool to rotate the shaft portion 211. The engagement recess portions 212A are recessed in the axis AX2 direction and have a starting point at a base end portion 212C, which is a part of a cylindrical circumferential wall 212B that constitutes the large diameter portion 212. Four engagement recess portions 212A are provided at intervals in the circumferential direction of the large diameter portion 212. In the present embodiment, the intervals are preferably equal intervals (every 90 degrees).

Here, the shaft portion 211 is not limited to the above configuration and all sections may have the same diameter, or sections with different diameters may be mixed in other modes.

As shown by the cross-sectional view of Fig. 2(B), the shaft portion 211 includes a shaft hole 218 extending in the axis AX2 direction. In the present embodiment, the shaft hole 218 penetrates the shaft portion 211 (the large diameter portion 212, the tapered portion 213, and the small diameter portion 214), but the shaft hole 218 is not limited to this. For example, the shaft hole 218 may extend from an opening 211D on the base end side of the shaft portion 211 to a middle point of the small diameter portion 214. The diameter of the shaft hole 218 is approximately the same from the base end to the tip of the shaft portion 211 but is not limited to this.

In addition, the small diameter portion 214 includes a cylindrical circumferential wall 214A that surrounds the shaft hole 218. As shown in Fig. 2(B), the small diameter portion 214 includes a hole portion 215 penetrating the circumferential wall 214A. The hole portion 215 is preferably provided in the vicinity of a missing portion 223, which will be discussed later. The hole portion 215 is provided in one or more places. In the present embodiment, four hole portions 215 are provided at equal intervals (every 90 degrees) in the circumferential direction around the axis of the shaft portion 211. Specifically, since the shaft hole 218 needs to communicate with the outside through the hole portions 215, the length of the shaft hole 218 extends from the opening 211D on the base end side to at least the position where the hole portions 215 are provided. The hole portions 215 will be described in detail later.

### <Screw engagement portion>

The screw engagement portion 221 will be described below with reference to Figs. 2 to 4. As shown in Figs. 2(A), 3(A), and 3(B), the screw engagement portion 221 includes a plurality of unit screw threads 222, which are continuously formed on the circumferential surface of the shaft portion 211 around the axis thereof in a spiral shape. As a result, screw grooves 224 in the same spiral shape are formed between unit screw threads 222 adjacent in the axis AX2 direction. Here, as shown with the cross section taken in the axis AX2 direction in Fig. 2(B), the unit screw threads 222 are formed such that tapered (saw blade-like) screw threads, with their widths gradually decreasing from a surface 211A of the shaft portion 211 toward the outside of the shaft portion 211 in a radial direction R, make a round (rotate 360 degrees) spirally around the circumferential surface of the shaft portion 211. As shown in Fig. 2(A), in the present embodiment, nine unit screw threads 222 are provided. Among these, a fourth unit screw thread (hereinafter referred to as a specific unit screw thread) 222A counting from the tip side of the shaft portion 211 has the missing portion 223. Note that the specific unit screw thread may be any unit screw thread 222. In other words, an n-th unit screw thread counting from the tip can be defined as the specific unit screw thread.

As shown in Figs. 3(A) to 3(C), the missing portion 223 is formed by removing part of a specific unit screw thread 222A. As shown in Fig. 3(C), internal spaces of a pair of screw grooves 224A and 224B adjacent across the specific unit screw thread 222A face (are opposed to) each other through the missing portion 223. In other words, one screw groove 224A can face a fifth ((n+1)-th) unit screw thread 222D through the missing portion 223, and the other screw groove 224B can face a third ((n-1)-th) unit screw thread 222C through the missing portion 223. As shown in the schematic view of Fig. 3(D), in the present embodiment, the missing portion 223 includes a first missing region B1 formed into a notched or hole shape because of the absence of at least part of a section (missing section) A in a round direction of the fourth (n-th) specific unit screw thread 222A. Specifically, the first missing region B1 is formed in at least part of a region extending from the root (the bottom position of the screw groove) to a top portion 222B of the specific unit screw thread 222A in the missing section A. The first missing region B1 provides communication between the internal spaces of the pair of screw grooves 224A and 224B in the axial direction. Here, the round direction refers to a direction (spiral direction) in which the unit screw threads are provided around the circumferential surface of the shaft portion 211.

Here, the missing region of the missing portion 223 is not limited to the configuration of the first missing region B1. The missing section A of the specific unit screw thread 222A may be configured such that some part thereof maintains the continuous state of the specific unit screw thread 222A, while the remaining part may be removed. For example, a second missing region B2 of the missing portion 223 in Fig. 4(A) is a part of the missing section A (part of the screw thread in a height direction H) that starts from the root of the specific unit screw thread 222A (the bottom position of the screw groove) and extends to a portion before the top portion 221B of the specific unit screw thread 222A. Moreover, for example, a third missing region B3 of the missing portion 223 in Fig. 4(B) is part of the missing section A that starts from the top portion 221B of the specific unit screw thread 222A and extends to a portion before the root of the specific unit screw thread 222A (the bottom position of the screw groove). Furthermore, for example, a fourth missing region B4 of the missing portion 223 in Fig. 4(C) is part of the missing section A that starts from the portion blow the top portion 221B of the specific unit screw thread 222A in the height direction H and extends to a portion above the root (the bottom position of the screw groove) in the height direction H. In addition, the missing portion 223 may be configured with a mixture of the modes in Figs. 4(A) to (C) shown above. In any of these missing portions 223, the internal spaces of the pair of screw grooves 224A and 224B, which are adjacent across the specific unit screw thread 222A, face (are opposed to) each other through the missing portion 223. Note that the first missing region B1 to the fourth missing region B4 are all in a square (or partially fan) shape, though they are not limited to the square shape, and any shape is adoptable.

As shown in Figs. 3(A) and 3(C), the third ((n-1)-th) unit thread 222C and the fifth ((n+1)-th) unit thread 222D, which are adjacent to the specific unit thread 222A in the axis AX2 direction, do not have a missing region (corresponding region) corresponding to the missing portion 223 of the fourth (n-th) specific unit thread 222A. Here, the third ((n-1)-th) unit screw thread 222C and the fifth ((n+1)-th) unit screw thread 222D may have a missing portion in regions other than the corresponding region described above.

### <Positional relation between missing portion and hole portion>

The positional relation between the missing portion 223 and the hole portion 215 will be described with reference to Figs. 4 and 5. As shown in Fig. 5(A), the hole portion 215 in the present embodiment includes a long hole region 228 extending across the pair of screw grooves 224A and 224B and the missing portion 223. Here, the hole portion 215 becomes the long hole itself, with the axis AX2 direction being the longitudinal direction. Since the hole portion 215 is a long hole, it is possible to swiftly insert an intraosseous injection agent in the axial direction even with a small number of holes and arrangement and to maintain the strength of the lag screw. As shown in Fig. 5(A), in a plan view of the missing portion 223 and the hole portion 215 from the radial direction R of the shaft portion 211 (the direction perpendicular to the page surface), a region corresponding to the missing portion 223 is defined as a missing portion exclusive region R1, a region of the screw groove 224A adjacent to the missing portion exclusive region R1 is defined as a first screw groove region R2, and a region of the screw groove 224B adjacent to the missing portion exclusive region R1 is defined as a second screw groove region R3. In this case, the long hole region 228 extends across the first screw groove region R2, the missing portion exclusive region R1, and the second screw groove region R3. Note that the missing portion exclusive region R1 includes any one of the first missing region B1 to the fourth missing region B4 described above, or any combination thereof. The first screw groove region R2 and the second screw groove region R3 are also arranged in the vicinity of the missing portion exclusive region R1 in the axis AX2 direction. It is also preferable that an extending direction (longitudinal direction) of the long hole region 228 is approximately parallel to the axis AX2 direction.

Here, in the case where the hole portion 215 becomes the long hole region 228 as shown in Fig. 5(A), the long hole region 228 is structured to be continuous (continuing) to the first missing region B1 or the second missing region B2 shown in Figs. 3(D) and 4(A).

Here, the hole portion 215 may not include the long hole region 228 extending across the pair of screw grooves 224A and 224B and the missing portion 223. For example, as shown in Fig. 5(B), the hole portion 215 may be constituted of a plurality of holes provided in each of the first screw groove region R2 and the second screw groove region R3, without extending across the missing portion exclusive region R1.

As shown in Figs. 5(C) and 5(D), the hole portion 215 may extend across the missing portion exclusive region R1 and the first screw groove region R2, or may extend across the missing portion exclusive region R1 and the second screw groove region R3. The mode in Figs. 5(C) and 5(D) is combined with the mode of the third missing region B3 shown in Fig. 4(B). In this case, it is preferable to form, on the specific unit screw thread 222A, a recessed surface 219 that is recessed in its thickness direction E (approximately parallel to the axis AX2 direction) so that the entire missing portion exclusive region R1 of the hole portion 215 is open to the outside. As shown in Fig. 5(D), the recessed surface 219 preferably extends so as to be continuous to the third missing region B3 with the surface 211A of the shaft portion 211 as a starting point. The same is true for the fourth missing region B4.

### <Missing portion and hole portion in shaft portion>

An arrangement mode of the missing portions 223 and the hole portions 215 in the shaft portion 211 will be described with reference to Figs. 6 and 7. Fig. 6(A) is an enlarged view of the screw engagement portion 221 provided in the small diameter portion 214 of the osteosynthesis member (lag screw) 201. Fig. 6(B) is a cross-sectional view of Fig. 6(A) taken along an F-F arrow line.

As shown in Fig. 6(B), in the present embodiment, four missing portions 223 and four hole portions 215 are provided at intervals in a circumferential direction C of the shaft portion 211. It is preferable that the missing portions 223 and the hole portions 215 be provided at equal intervals. In present embodiment, the plurality of missing portions 223 and the hole portions 215 are provided so that the adjacent missing portions 223 and hole portions 215 have a 90 degree phase difference in the circumferential direction C of the shaft portion 211. Note that the circumferential direction C is a direction around the axis of the shaft portion 211.

Here, the number of the missing portions 223 and the hole portions 215 may be one or more. When there are two or more missing portions 223 and hole portions 215, the missing portions 223 and the hole portions 215 may be provided at unequal intervals or, equal and unequal intervals may be mixedly present, as long as the missing portions 223 and the hole portions 215 are spaced apart in the circumferential direction C of the shaft portion 211.

Furthermore, in the present embodiment, the four missing portions 223 are provided in a single specific unit screw thread 222A. The hole portions 215 are provided at the positions corresponding to the respective missing portions 223. Therefore, the four missing portions 223 and four hole portions 215 are provided at approximately the same positions in the axis AX2 direction. Note that strictly speaking, a single specific unit screw thread 222A is spirally provided to the shaft portion 211, and therefore, the positions of the respective missing portions 223 and the respective hole portions 215 are shifted from each other in the axis AX2 direction by the lead of the spiral.

When the osteosynthesis member (lag screw) 201 is configured as described above, six-view drawings of the osteosynthesis member (lag screw) 201 are expressed as shown in Figs. 7(A) to 7(E). Incidentally, Fig. 7(A) is a plan view of the osteosynthesis member (lag screw) 201. Fig. 7(B) includes, in order from the left side, a left side view, a front view, and a right side view of the osteosynthesis member (lag screw) 201. Fig. 7(C) is a bottom view of the osteosynthesis member (lag screw) 201. Fig. 7(D) is a rear view of the osteosynthesis member (lag screw) 201. Fig. 7(E) is a perspective view of the osteosynthesis member (lag screw) 201.

As shown in Fig. 7, the arrangement of the four hole portions 215 of the osteosynthesis member 201 in the circumferential direction C has a phase difference of 45 degrees with respect to the arrangement of the four engagement recess portions 212A, which are formed at the base end of the shaft portion 211 in the circumferential direction C. Grasping this correspondence relation enables the engagement recess portions 212A to serve as a marker for the arrangement of the hole portions 215.

### <Surgical method using osteosynthesis implement>

The osteosynthesis implement 100 of the present embodiment is applied to the treatment of fractures (femoral neck fractures) in the vicinity of the bone head of the femur, for example. Hereinafter, an example of a surgical method (procedure) using the osteosynthesis implement 100 will be described with reference to Figs. 8 and 9 as well as the drawings described above.

First, as shown in Fig. 1(B), an entry hole is formed at an upper end portion 501 on the side of the bone head 502 in the femur 500 using an awl or the like. Then, the entry hole is widened using a drilling device such as a drill and a reamer to subject the cortical bone to an opening treatment. As a result, an opening is formed at the end portion 501. The opening of the cortical bone is made to communicate with the medullary cavity of the femur 500.

Then, the tip of the intramedullary nail 101 is introduced through the opening formed at the end portion 501, so that the intramedullary nail 101 is inserted into the medullary cavity along an axis line of the femur 500.

Then, an unillustrated intramedullary nail mounting device (target device) is connected to the upper end T (the top portion, the base end) of the intramedullary nail 101, which is exposed from the femur 500. A tip (a retainer) of the intramedullary nail mounting device is engaged with a notch portion 1012 provided at the upper end T of the intramedullary nail 101, and then a fixing bolt not particularly illustrated is screwed into the female screw 106 so as to couple the intramedullary nail mounting device to the intramedullary nail 101. Then, a guide pin (not illustrated) is inserted from the outside of the body along the axis line of the transverse hole 104 in accordance with a guide (guide) provided in advance in the intramedullary nail mounting device. The tip of the guide pin crosses a fracture line 505 and reaches the cortical bone of the bone head 502.

The guide pin is then used to guide the drilling device such as a drill and a reamer. The drilling device creates a bone hole 503 along the axis line of the transverse hole 104 for the femur 500.

Next, after the drilling device is extracted, the osteosynthesis member (lag screw) 201 is guided using the guide pin. As a result, the osteosynthesis member 201 passes through the transverse hole 104 and is screwed into the bone hole 503 formed in the femur 500. At this time, the screw engagement portion 221 of the osteosynthesis member 201 reaches the cortical bone of the bone head 502 beyond the fracture line 505.

As a result, the osteosynthesis member 201 is fixed to the bone head 502, and the osteosynthesis member 201 is further pulled toward the intramedullary nail 101, and at this time, regions around the fracture line 505 of the femur 500 are aligned, and fracture sites on both sides, which are in contact with each other at the fracture line 505, are set to adhere to each other.

In this state, the set screw 451 is screwed into the shaft hole 105 from the base end of the intramedullary nail 101, and the tip 451A of the set screw 451 is brought into contact with the groove portions 216 of the osteosynthesis member 201. As a result, the osteosynthesis member 201 is fixed to the intramedullary nail 101.

Next, an injector 301 shown in Fig. 8 is used to feed an intraosseous injection agent into the bone head 502 through the osteosynthesis member (lag screw) 201. In the present invention, a combination of the injector 301 and the osteosynthesis implement 100 is called an osteosynthesis implement set. The intraosseous injection agent is a material used in the treatment of bone, such as reinforcing bone tissues, and is preferably a pasty agent. Examples of the intraosseous injection agent may include calcium phosphate-based bone prosthetic materials, and bone cement composed of polymethyl methacrylate.

The injector 301, which allows injection of the intraosseous injection agent into bone, includes a syringe 302 having a material filling space 302A inside, a plunger 303, and an injection nozzle 304 to be connected to the syringe 302 as shown in Fig. 8. The injection nozzle 304 has an external diameter that allows insertion into the shaft hole 218, and also has an injection port 305 in the vicinity of the tip. The plunger 303 includes a mobile body 306, and a pressing portion 307 that presses the mobile body 306 to move the mobile body 306 in the axial direction of the syringe 302.

The mobile body 306 includes a tip portion 306A retained in the syringe 302 so as to be movable in the axial direction of the syringe 302, while the outer circumferential surface of the mobile body 306 is in contact with the inner circumferential surface of the syringe 302, and also includes a base end portion 306B continuing to the tip portion 306A. The base end portion 306B faces the pressing portion 307 in the axial direction of the syringe 302.

The pressing portion 307 includes a screw portion 307A and a screw engagement portion 307B. The screw portion 307A is screwed to the screw engagement portion 307B. When the screw portion 307A is rotated forward, the screw portion 307A moves in the axial direction of the syringe 302 so as to approach the mobile body 306 (base end portion 306B). When the screw portion 307A is rotated backward, the screw portion 307A moves in the axial direction of the syringe 302 so as to be separated from the mobile body 306 (the base end portion 306B) .

When the screw portion 307A is rotated forward while the intraosseous injection agent is filled in the material filling space 302A, the screw portion 307A approaches the base end portion 306B. When the screw portion 307A presses the base end portion 306B, the tip portion 306A moves, together with the base end portion 306B, in the direction of pressing the intraosseous injection agent. As a result, the intraosseous injection agent is extruded into the injection nozzle 304 and extruded to the outside through the injection port 305.

To feed the intraosseous injection agent into the bone head 502 using the injector 301 configured as described above, the injection nozzle 304 is first inserted into the shaft hole 218 of the osteosynthesis member 201 that is fixed to the intramedullary nail 101 as shown in Fig. 9(A). It is preferable that the outer diameter of the injection nozzle 304 be approximately identical to or slightly smaller than the diameter of the shaft hole 218. This is to make it difficult for the intraosseous injection agent to enter into a gap between the shaft hole 218 and the injection nozzle 304.

Then, as shown in Fig. 9(B), the entire injector 301 is rotated in the circumferential direction C to align the injection port 305 with the hole portions 215 of the osteosynthesis member 201. It is preferable to place a marker on each of the osteosynthesis member 201 and the injector 301 so that the relative position of the hole portions 215 of the osteosynthesis member 201 and the injection port 305 of the injector 301 (relative phase difference in the circumferential C) is recognizable from the outside. In the present embodiment, the markers of the hole portions 215 of the osteosynthesis member 201 are the engagement recess portions 212A of the large diameter portion 212 in the shaft portion 211 as shown in Figs. 8 and 9(B). The marker of the injector 301 is a marking 309 provided on the outer circumferential surface of the injection nozzle 304 (or the syringe 302), as shown in Figs. 8 and 9(B). In addition, inside the shaft portion 211, a stopper 217 may be provided which comes into contact with the tip of the injection nozzle 304 in the vicinity of the tip of the shaft portion 211 to position the injection nozzle 304 in the axis AX direction. When a practitioner inserts the injection nozzle 304 until it touches the stopper 217, and then rotates and positions the injector 301 so that the marking 309 is positioned in a middle position of a pair of adjacent engagement recess portions 212A (with a 45 degree phase difference), the position of the hole portion 215 of the osteosynthesis member 201 coincides with the position of the injection port 305. When a syringe 302 filled with the intraosseous injection agent is pressed with the plunger 303 in this state, the intraosseous injection agent is extruded into the bone head 502 through the injection nozzle 304, the injection port 305, and the hole portion 215 in this order. This operation is repeated for all the hole portions 215. Here, as shown in Fig. 9(B), it is also preferable to provide, as a marker on the side of the injector 301, a marking 310 indicating the depth of penetration into the hole portion 215 of the osteosynthesis member 201 in the axis AX2 direction.

As shown in Figs. 10(A), 11(A), and 12(A), when the intraosseous injection agent is extruded from the hole portion 215, the intraosseous injection agent spreads from the long hole region 228 to the missing portion exclusive region R1, the first screw groove region R2, and the second screw groove region R3. Then, when the pressing continues, the intraosseous injection agent spreads in the direction along the screw groove 224A and the screw groove 224B beyond the first screw groove region R2 and the second screw groove region R3, and also spreads in the height direction H of the screw groove 224A and the screw groove 224B as shown in Figs. 10(B), 10(C), 11(B), 11(C), and 12(B). In this case, in a plan view of the missing portion 223 and the hole portion 215 from the radial direction of the shaft portion 211 (the direction perpendicular to the page surface) as shown in Figs. 10(B) and 10(C), the intraosseous injection agent spreads in an H-shape. Viscous resistance of the intraosseous injection agent when spreading in the H-shape increases the pressure of the intraosseous injection agent.

Finally, as shown in Fig. 12(C), the screw groove 224A and the screw groove 224B are filled with the intraosseous injection agent, while at the same time, as shown in Fig. 12(D), the intraosseous injection agent further moves to radially outside the screw groove 224A and the screw groove 224B, and enters into the bone head 502 adjacent to the screw engagement portion 221. Then, the intraosseous injection agent further spreads along the (unit) screw groove 224A and the (unit) screw groove 224B, and gradually spreads to a (unit) screw groove 224C continuing to the (unit) screw groove 224A on the tip side in the axis AX2 direction and to a (unit) screw groove 224D continuing to the screw groove 224B on the base end side. The same applies to the other screw grooves.

In addition, the plurality of hole portions 215 and missing portions 223 are concentrated in the specific unit screw thread 222A, which prevents wasteful dispersion of the intraosseous injection agent. Accordingly, the intraosseous injection agent spreads around the specific unit screw thread 222A, and when the intraosseous injection agent is hardened, the osteosynthesis member 201 and the bone head are firmly fixed to each other.

Here, as shown in Figs. 13(A) and 13(B), when the osteosynthesis member 201 rotates in the circumferential direction C, the intraosseous injection agent, filled in the region (the missing portion exclusive region R1) corresponding to the missing portion 223, comes into contact with two surfaces 229 that face (are opposed to) each other across the missing portion 223 of the specific unit screw thread 222A and restricts rotation of the osteosynthesis member 201. In other words, the intraosseous injection agent filled in the region (the missing portion exclusive region R1) corresponding to the missing portion 223 functions as a rotation restriction portion that restricts the rotation of the osteosynthesis member 201.

### <Advantages of osteosynthesis member>

As shown in Figs. 14(A) and 14(B) showing a comparative example, a case is examined in which the intraosseous injection agent is extruded through the hole portions 215 provided in the screw grooves 224 and is filled along the screw grooves 224 without crossing the unit screw threads 222 so that the osteosynthesis member 201 is constrained between the intraosseous injection agent and the bone head. In this case, as shown in Figs. 14(C) and 14(D), when a gap region 900 is generated between the intraosseous injection agent and the screw grooves 224 or between the intraosseous injection agent and the shaft portion 211 due to some cause, the force of constraining the osteosynthesis member 201 is weakened. As a result, when vibrations are applied to the osteosynthesis member 201 due to the motions of the patient, the osteosynthesis member 201 rotates relatively to the bone head 502, which weakens the force to fix the fractured portion.

On the other hand, according to the osteosynthesis member 201 of the present embodiment, even when the gap region 900 is generated between the intraosseous injection agent and the screw grooves 224A and 224B or between the intraosseous injection agent and the shaft portion 211, the intraosseous injection agent filled in the regions corresponding to the missing portions 223 functions as the rotation restriction portion, as shown in Figs. 13(C) and 13(D). As a result, even when vibrations are applied to the osteosynthesis member 201 due to the motions of the patient, the relative rotation of the osteosynthesis member 201 and the bone head 502 is restricted, and the force to fix the fractured portion is maintained.

Moreover, in the case where the intraosseous injection agent is filled along the screw grooves 224, and the osteosynthesis member 201 is firmly fixed between the intraosseous injection agent and the bone head as shown in Figs. 14(A) and 14(B) that show a reference example, at the time of removing the osteosynthesis member 201, it is necessary to rotate the osteosynthesis member 201 in a loosening direction so as to slide the osteosynthesis member 201, with respect to the intraosseous injection agent solidified, along the screw groove 224. This causes a frictional force that can destroy the bone head.

Meanwhile, at the time of removing the osteosynthesis member 201 of the present embodiment as shown in Figs. 13(A) and 13(B), the rotation of the osteosynthesis member 201 allows the surface 229 of the specific unit screw thread 222A to apply a strong local pressure to the intraosseous injection agent solidified in the missing portions 223, to thereby destroy the pressure received portions. When part of the intraosseous injection agent is destroyed, cracks or the like spread throughout the entire intraosseous injection agent, which makes it possible to easily rotate the osteosynthesis member 201 and remove the osteosynthesis member 201.

As shown in Fig. 15, which is a modification of the present embodiment, it is also possible to provide the missing portions 223, which are in phase with the missing portions 223 of the specific unit screw thread 222A, in each of specific unit screw threads 222F and 222G adjacent to the specific unit screw thread 222A. In other words, the plurality of missing portions 223 adjacent in the axis AX2 direction are provided continuously in the axis AX2 direction in which the missing portions 223 face each other. In this case, the intraosseous injection agent extruded from the hole portions 215 flows both in a continuous direction P of the missing portions 223 and in the direction along the screw grooves 224. As compared with the case of Fig. 13, there are more routes for the intraosseous injection agent to spread, which requires a larger liquid amount and a longer filling time for the intraosseous injection agent to sufficiently spread radially outward (to the bone head side) from the screw grooves 224. However, there is also an advantage that the intraosseous injection agent easily spreads in the axial direction AX2.

On the other hand, the osteosynthesis member 201 shown in Figs. 12 and 13 does not have missing regions (corresponding regions), corresponding to the missing portions 223 of the specific unit screw thread 222A, in the unit screw threads 222C and 222D which are adjacent to the specific unit screw thread 222A in the axis AX2 direction. In other words, the route for the intraosseous injection agent to spread is actively limited to the pair of screw grooves 224A and 224B. This results in an advantage that the intraosseous injection agent flows along the screw grooves 224A and 224B of the osteosynthesis member 201, and at an early stage, the osteosynthesis member 201 spreads radially outward and sufficiently reaches the surrounding bone head tissues. Since the osteosynthesis member 201 is locally concentrated and solidified inside the bone head, the strength of the bone head tends to increase.

### <Second embodiment>

Next, a second embodiment of the present invention will be described with reference to Fig. 16. The osteosynthesis implement in the present embodiment is different in arrangement mode of the missing portions 223 and the hole portions 215 in the shaft portion 211 from that of the first embodiment.

Figs. 16(A) to 16(D) show, in this order, a plan view of a modification of the osteosynthesis member (lag screw) 201 in the present embodiment, a front view of the osteosynthesis member (lag screw) 201 in the present embodiment, a bottom view of the osteosynthesis member (lag screw) 201 in the present embodiment, and a rear view of the osteosynthesis member (lag screw) 201 in the present embodiment. In the osteosynthesis member (lag screw) 201 shown in Figs. 16(A) to 16(D), the respective missing portions 223 are provided in separate specific unit screw threads 222A. Furthermore, as in the case of the first embodiment, the plurality of missing portions 223 are arranged at the positions not facing each other in the axial direction (the positions out of phase in the circumferential direction).

When the respective missing portions 223 and the hole portions 215 are provided in the separate specific unit screw threads 222A as in this osteosynthesis member (lag screw) 201, the shaft portion 211 and the screw engagement portion 221 therearound can advantageously obtain uniform strength in the axis AX2 direction.

In the second embodiment, the case has been illustrated in which each of the specific unit screw threads 222A includes one missing portion 223 and one hole portion 215. However, the number of the missing portions 223 and the hole portions 215 is not particularly limited.

### <Third embodiment>

Next, an osteosynthesis implement of a third embodiment of the present invention will be described with reference to Fig. 17. The osteosynthesis implement in the present embodiment is different in the mode of the missing portions 223 and the hole portions 215 from that of the first embodiment. In the present embodiment, as shown in Fig. 17(A), the plurality of hole portions 215 and missing portions 223 are arranged at the positions in phase with each other in the circumferential direction and at a distance from each other in the axis AX2 direction. The two hole portions 215 arrayed in series and their corresponding missing portions 223 form pairs, respectively, and are spaced apart in the circumferential direction of the shaft portion 211.

Here, as shown in Fig. 17(A), between the two missing portions 223 (and the hole portions 215) that are in the same phase in the circumferential direction, at least one unit screw thread 222 is present which does not have a missing region that is in phase with the two missing portions 223. In Fig. 17(A), there is only one unit screw thread 222 that does not have a missing region corresponding to the two missing portions 223, and a plurality of unit screw threads 222 may be present as shown in Fig. 17(B). In addition, as compared with Fig. 17(A), the width (circumferential width) of the hole portions 215 and the missing portions 223 in the circumferential direction of the shaft portion 211 is set to be larger in Fig. 17 (B). However, the width is not particularly limited. Note that the region (phase width) occupied by one missing portion 223 in the circumferential direction is preferably 40 degrees or less, desirably 30 degrees or less, and more desirably 20 degrees or less.

### <Fourth embodiment>

Next, an osteosynthesis implement of a fourth embodiment of the present invention will be described with reference to Fig. 18. In the osteosynthesis implement of the present embodiment, the hole portion 215 and the missing portion 223 are formed in each of the plurality of (three in this case) specific unit screw threads 222A, 222C and 222D which are adjacent in the axis AX2 direction. The missing portion 223 formed in the specific unit screw thread 222 (222C, 222D) is located in the region that is shifted in the round direction from the region facing the missing portion 223 of the specific unit screw thread 222A in the middle.

The hole portion 215 formed in the specific unit screw thread 222A extends across the screw grooves 224A (the first screw groove region R2), 224B (the second screw groove region R3), and the missing portion exclusive region R1 corresponding to the specific unit screw thread 222A. The hole portion 215 formed in the specific unit screw thread 222C extends across the screw grooves 224C (the third screw groove region R4), 224A (the first screw groove region R2), and the missing portion exclusive region R1 corresponding to the specific unit screw thread 222C. The hole portion 215 formed in the specific unit screw thread 222D extends across the screw grooves 224D (the fourth screw groove region R5), 224B (the second screw groove region R3), and the missing portion exclusive region R1 corresponding to the specific unit screw thread 222D. In this way, the plurality of missing portions 223 are formed in a so-called labyrinth form for the plurality of (three in this case) adjacent specific unit screw threads 222A, 222C, and 222D.

In the osteosynthesis implement of the present invention, the case where the screw engagement portion 221 is a single-thread screw is illustrated. However, without being limited thereto, the osteosynthesis implement is also applicable to multi-thread screws with two or more threads.

The osteosynthesis implement of the present invention is not limited to the embodiments described above, and various modifications can be made without departing from the gist of the present invention. Various combinations of the respective component members in the respective embodiments are also included in the scope of the present invention.

### Reference Signs List

10 intramedullary nail aid
100 osteosynthesis implement
101 intramedullary nail (nail)
104 transverse hole
201 osteosynthesis member (lag screw)
211 shaft portion
212A engagement recess portion
215 hole portion
216 groove portion
218 shaft hole
221 screw engagement portion
222, 222C, 222D unit screw thread
222A, 222E specific unit screw thread
223 missing portion
224, 224A to 224D screw groove
228 long hole region
301 injector
302 syringe
303 plunger
304 injection nozzle
305 injection port

## Claims

1. An osteosynthesis implement (100) to be inserted into an intramedullary nail (101), the osteosynthesis implement (100) including a shaft portion (211), and a screw engagement portion (221) formed so that a plurality of unit screw threads (222, 222A, 222C, 222D, 222E, 222F, 222G), provided around a circumferential surface of the shaft portion (211), are continuous in a spiral shape, the osteosynthesis implement (100) **being characterized by** comprising:
a shaft hole (218) extending in an axial direction inside the shaft portion (211);
a missing portion (223) formed by removing part of a specific unit screw thread (222A, 222C, 222D, 222F, 222G) so that internal spaces of a pair of screw grooves (224, 224A, 224B, 224C, 224D), adjacent across the specific unit screw thread (222A, 222C, 222D, 222F, 222G), face each other; and
a hole portion (215) that is opened so that each screw groove of the pair of screw grooves (224, 224A, 224B, 224C, 224D) in a vicinity of the missing portion (223) communicates with the shaft hole (218).

2. The osteosynthesis implement (100) according to claim 1, **characterized in that**
the hole portion (215) includes a long hole region (228) formed so as to extend across both screw grooves in the pair thereof (224, 224A, 224B, 224C, 224D).

3. The osteosynthesis implement (100) according to claim 2, **characterized in that**
the long hole region (228) extends in a direction that is approximately parallel to a length direction of the shaft portion (211) .

4. The osteosynthesis implement (100) according to any one of claims 1 to 3, **characterized in that**
the unit screw threads (222, 222C, 222D, 222E, 222F) adjacent to the specific unit screw thread (222A, 222C, 222D) do not have a missing region (B1 to B4) facing the missing portion (223) of the specific unit screw thread (222A, 222C, 222D).

5. The osteosynthesis implement (100) according to any one of claims 1 to 4, **characterized in that**
the missing portion (223) and the hole portion (215) are provided in a plurality of places in a circumference direction of the shaft portion (211).

6. The osteosynthesis implement (100) according to any one of claims 1 to 5, **characterized in that**
the missing portion (223) and the hole portion (215) are provided in four places at approximately equal intervals around an axis (AX2) of the shaft portion (211).

7. The osteosynthesis implement (100) according to any one of claims 1 to 6, **characterized in that**
the missing portion (223) and the hole portion (215) are not provided in unit screw threads (222, 222C, 222D, 222E, 222F) other than the specific unit screw thread (222A, 222C, 222D).

8. The osteosynthesis implement (100) according to claim 1, **characterized in that**
the missing portion (223) does not extend across two or more of the unit screw threads (222, 222A, 222C, 222D, 222E, 222F, 222G).

9. An osteosynthesis implement (100) to be inserted into an intramedullary nail (101), the osteosynthesis implement (100) including a shaft portion (211) having a shaft hole (218), and a screw thread (222, 222A, 222C, 222D, 222E, 222F, 222G) provided at a tip of the shaft portion (211), **characterized in that:**
the shaft portion (211) includes a hole portion (215) of an elongated shape extending in a direction approximately parallel to a length direction of the shaft portion (211); and
the hole portion (215) penetrates from a surface of the shaft portion (211) to the shaft hole (218).

10. An osteosynthesis implement (100) set, comprising:
the osteosynthesis implement (100) according to any one of claims 1 to 9; and
an injector (301) configured to allow injection of an intraosseous injection agent into a bone, **characterized in that**
the injector (301) includes
an injection nozzle (304) configured to be insertable into the shaft hole (218), and
a single injection port (305) provided so as to correspond to the hole portion (215) at a tip of the injection nozzle (304).

11. An osteosynthesis member set, comprising:
an osteosynthesis member (201) to be inserted into an intramedullary nail (101), the osteosynthesis member (201) including a shaft portion (211), a screw thread provided spirally around a circumferential surface of the shaft portion (211), and a shaft hole (218) extending in an axial direction inside the shaft portion (211); and
an injector (301) configured to allow injection of an intraosseous injection agent into a bone, **characterized in that:**
the osteosynthesis member (201) includes
a missing portion (223) provided in a unit screw thread (222, 222A, 222C, 222D, 222F, 222G) so that internal spaces of a pair of screw grooves (224, 224A, 224B, 224C, 224D), adjacent across the unit screw thread (222, 222a, 222c, 222d, 222e), face each other, the unit screw thread (222A, 222C, 222D, 222F, 222G) corresponding to one round of 360° of the screw thread (222, 222A, 222C, 222D, 222F, 222G),
a hole portion (215) provided in the shaft portion (211) so as to communicate with the missing portion (223), and
an osteosynthesis member-side positioning portion;
the injector (301) includes
an injection nozzle (304) configured to be insertable into the shaft hole (218) of the osteosynthesis member (201),
a single injection port (305) provided at a tip portion of the injection nozzle (304), and
an injector-side positioning portion; and
the osteosynthesis member-side positioning portion and the injector-side positioning portion are used to position the injector (301) with respect to the osteosynthesis member (201) to allow the injection port (305) to correspond to the hole portion (215).

12. The osteosynthesis member set according to claim 11, **characterized in that:**
the hole portion (215) is provided in a plurality of places in a circumference direction of the shaft portion (211); and
the osteosynthesis member-side positioning portion is provided in a plurality of places corresponding to the hole portions (215) in the circumference direction of the shaft portion (211).

13. The osteosynthesis member set according to claim 12, **characterized in that**
the hole portion (215) is provided at four places along the unit screw thread (222A, 222C, 222D, 222F, 222G) at approximately 90° intervals around an axis (AX2) of the shaft portion (211).

14. The osteosynthesis member set according to any one of claims 11 to 13, **characterized in that:**
the osteosynthesis member (201) includes, at a base end portion (212C) of the shaft portion (211), an engagement recess portion (212A) that is fitted with an instrument that rotates the osteosynthesis member (201); and
the engagement recess portion (212A) functions as the osteosynthesis member-side positioning portion.

15. The osteosynthesis member set according to any one of claims 11 to 14, **characterized in that**
the injection port (305) is a long hole extending in an axial direction of the injection nozzle (304) so as to cover the hole portion (215).
